## Europäisches Patentamt
## European Patent Office
## Office européen des brevets

(19)

(11) Veröffentlichungsnummer: **0 049 500**
**B1**

(12)

# EUROPÄISCHE PATENTSCHRIFT

(45) Veröffentlichungstag der Patentschrift:
**22.02.84**

(21) Anmeldenummer: **81107867.4**

(22) Anmeldetag: **24.04.80**

(60) Veröffentlichungsnummer der früheren Anmeldung nach Art. 76 EPÜ:

(51) Int. Cl.³: **C 07 C 141/16,** C 07 C 103/52 //
A61K37/02, A61K37/24

(54) **Tyrosinderivate, Verfahren zu ihrer Herstellung und ihre Verwendung bei der Synthese von Peptiden.**

(30) Priorität: **30.04.79 DE 2917603**
**25.10.79 DE 2943132**

(43) Veröffentlichungstag der Anmeldung:
**14.04.82 Patentblatt 82/15**

(45) Bekanntmachung des Hinweises auf die Patenterteilung:
**22.02.84 Patentblatt 84/8**

(84) Benannte Vertragsstaaten:
**AT CH DE FR GB LI SE**

(56) Entgegenhaltungen:
**DE - A - 1 800 129**
**DE - A - 1 935 402**
**DE - A - 2 022 623**
**DE - A - 2 751 026**

(73) Patentinhaber: **Max-Planck-Gesellschaft zur Förderung der Wissenschaften e.V., Bunsenstrasse 10, D-3400 Göttingen (DE)**

(72) Erfinder: **Wünsch, Erich, Dr. Prof., Midgardstrasse 16, D-8132 Tutzing (DE)**

(74) Vertreter: **Lederer, Franz, Dr. et al, Patentanwälte Dr. Franz Lederer Dipl.-Ing. Reiner F. Meyer-Roxlau Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

### Tyrosinderivate, Verfahren zu ihrer Herstellung und ihre Verwendung bei der Synthese von Peptiden

Tyrosin-O-sulfat wurde als Metabolit im Urin von Säugetieren und später auch als Eiweißbaustein in verschiedenen biologisch aktiven Peptiden und Proteinen, wie z. B. den Gastrinen, entdeckt. In den biologisch interessanten Peptidhormonen Cholecystokinin-Pankreocymin und Caerulein stellt dieser Aminosäurerest darüber hinaus eine »essentielle« Gruppierung für die biologische Aktivität dar. Die Synthese solcher biologisch aktiver tyrosin-O-sulfat-haltiger Peptide bzw. Proteine ist daher ein wichtiges Ziel der biochemischen, insbesondere peptid-chemischen Forschung und der Arzneimittelentwicklung.

Tyrosin-O-sulfat enthaltende biologisch aktive Peptide wurden bisher überwiegend durch nachträgliche Sulfatisierung des Phenolrests im Peptidverband mittels konzentrierter Schwefelsäure oder Pyridin/$SO_3$-Komplex hergestellt. Diese Methoden haben jedoch den Nachteil, daß in störendem Umfang Nebenreaktionen auftreten, wie Sulfonierung des Phenolkerns, des Indol- oder Imidazolrestes einerseits oder Umsetzungen mit der Methionin-Thioäther-Gruppierung, der Arginin-Guanido- und der primären Amidofunktion andererseits (J. Am. Chem. Soc., 68, 1024 und 1031 [1946]). Die zusätzlichen, leicht sulfatisierbaren Hydroxyl- und Aminofunktionen mußten geschützt werden.

Dieser Nachteil ließe sich vermeiden, wenn es gelänge, derartige aktive Peptide mit Tyrosin-O-sulfat als Startmaterial aufzubauen. Dadurch wurde es möglich, die durch die erwähnten Nebenreaktionen bedingten Beschränkungen bei der Peptidsynthese zu vermeiden. Es wurde zwar bereits über einen Versuch berichtet, mit Tyrosin-O-sulfat in Form des Kaliumsulfats derartige Peptide aufzubauen, jedoch wurden weder experimentelle Angaben hierüber noch die Resultate bekannt (Experientia, 28, 7 [1972]).

Mit Hilfe der erfindungsgemäßen Tyrosinderivate, nämlich der N-Acyl-tyrosin-O-sulfat-bariumsalze mit in der Peptidchemie üblichen Acylschutzgruppen, vorzugsweise des N-Carboxybenzoxy-tyrosin-O-sulfat-bariumsalzes gelingt es, die geschilderten Nachteile zu beseitigen und tyrosin-O-sulfat-halti-ge Verbindungen zur Verfügung zu stellen, welche sich als Startmaterial für die synthetische Herstellung von Peptiden eignen. Aufgrund ihrer Löslichkeitseigenschaften erleichtern sie die Isolierung der damit hergestellten Peptide.

Tyrosin-O-sulfat-bariumsalz besitzt die Formel H-Tyr-($SO_3Ba_{1/2}$)-OH, die genannte Carbobenzoxyverbindung die Formel B-Tyr-($SO_3Ba_{1/2}$)-O-$Ba_{1/2}$. In diesen Formeln bedeutet Tyr Tyrosin und B Carbobenzoxygruppe. Die verwendeten Abkürzungen für die Aminosäurereste entsprechen hier und in der folgenden Beschreibung den Regeln von Houben–Weyl, Methoden der organischen Chemie, 4. Auflage, Band 15/1, Seite 20, Verlag Thieme, Stuttgart (1974). Weitere, in der Peptidchemie übliche Acylschutzgruppen sind in Houben–Weyl, loc. cit., aufgeführt. Als Beispiele seien genannt N-t-Butyloxycarbonyl-, N-Fluorenyloxycarbonyl- und N-2-Nitrophenylsulfenyl-tyrosin-O-sulfat-bari-umsalz.

Erfindungsgemäß werden die N-Acyl-tyrosin-O-sulfat-bariumsalze dadurch hergestellt, daß man ein N-Acetyl-tyrosin in einem polaren organischen Lösungsmittel oder Lösungsmittelgemisch mit überschüssigem Pyridin-$SO_3$ umsetzt, die erhaltene Lösung mit Wasser extrahiert und aus der wäßrigen Phase das Bariumsalz des N-Acyl-tyrosin-O-sulfats durch Zusatz einer löslichen Bariumverbindung fällt. Bevorzugt wird als N-Acylgruppe die Carbobenzoxygruppe, welche hydrierend abgespalten werden kann.

Als polares organisches Lösungsmittel wird Pyridin oder ein Pyridin-Dimethylformamid-Gemisch bevorzugt. Es können jedoch auch andere schwach basische oder neutrale polare organische Lösungsmittel bzw. Gemische verwendet werden. Der Überschuß an Pyridin-$SO_3$-Komplex ist unkritisch, zweckmäßig wird eine 2- bis 6fache, vorzugsweise eine 3- bis 5fache Menge, bezogen auf $SO_3$-Äquivalent eingesetzt. Die Umsetzung wird zweckmäßig bei Temperaturen zwischen etwa 0°C und dem Siedepunkt des Lösungsmittels durchgeführt, bevorzugt werden Temperaturen zwischen etwa Zimmertemperatur und 80°C. Nach Beendigung der Umsetzung wird überschüssiger Pyridin-$SO_3$-Komplex durch Kühlung abgeschieden und aus der Lösung abgetrennt. Durch Einengung des Filtrats lassen sich zusätzliche Mengen an Komplex abtrennen. Anschließend wird mit Wasser verdünnt, Verunreinigungen werden mit organischen Lösungsmitteln, wie z. B. Essigester, extrahiert, und anschließend wird der wäßrigen Phase eine geeignete lösliche Bariumverbindung, vorzugsweise Bariumhydroxid, zugesetzt. Zweckmäßig wird dabei ein 2- bis 3facher Überschuß zugesetzt.

Durch $CO_2$-Zusatz läßt sich überschüssiges Bariumhydroxid abtrennen, wobei ein Sauerwerden der Lösung durch Zugabe eines schwachen Alkalis, vorzugsweise einer organischen Base, wie Pyridin, vermieden wird. Nach Entfernung des Niederschlags wird das acyl-gruppen-haltige Bariumsalz des Tyrosin-O-sulfats gewonnen. Es kann als solches für die Peptidsynthese verwendet oder in das Tyrosin-O-sulfat-bariumsalz überführt werden. Die Abspaltung der Acylgruppe erfolgt in der in der Peptidchemie üblichen Weise, z. B. bei der Carbobenzoxygruppe, durch Hydrierung in Gegenwart von Palladium als Katalysator, zweckmäßig in Dimethylformamid als Reaktionsmedium.

Die erfindungsgemäßen neuen Bariumsalze erweisen sich für den totalsynthetischen Aufbau tyrosin-O-sulfat-haltiger Peptide bzw. Proteine als besonders geeignet, da sie die Isolierbarkeit der damit hergestellten Peptidderivate positiv beeinflussen. Überraschenderweise ergab sich dabei, daß die üblichen Methoden der Peptidsynthese hierbei angewendet werden können, ohne daß die O-Sulfatgruppe abgespalten wird. Dies gilt unter anderem auch für die Abspaltung der Schutzgruppen, beispielsweise von Schutzgruppen auf tert.-Butanol-basis unter sauren Bedingungen. Dies war nicht zu erwarten, da bisher stets auf die Säureinstabilität des Tyrosin-O-sulfats hingewiesen wurde. Typische Beispiele für Peptidsynthesemethoden, die in Gegenwart der erfindungsgemäßen Verbindungen durchgeführt werden können, ohne letztere zu zersetzen, sind die Kondensation nach der Dicyclohexylcarbodiimid/1-Hydroxybenzotriazol-Methode (DCCD/HOBT-Methode), die Methoden über gemischte Anhydride mit z. B. Pivaloylchlorid, Chlorameisensäure-äthylester oder Isobutylester. Ein anderes Beispiel für eine geeignete Synthesemethode ist die Dicyclohexylcarbodiimid/N-Hydroxysuccinimid-Methode.

Die besondere Brauchbarkeit der erfindungsgemäßen Tyrosinderivate wird mit Hilfe des benzyloxycarbonylgruppenhaltigen Bariumsalzes am Beispiel der Synthese des Nonapeptids der Sequenz H-Arg-Asp-Tyr(SO$_3$H)-Thr-Gly-Trp-Leu-Asp-Phe-NH$_2$ demonstriert. Dabei wurde die erfindungsgemäße Bariumverbindung nach der DCCD/HOBT-Methode an H-Thr(tBu)-Gly-Trp-Leu-Asp(OtBu)-Phe-NH$_2$ aufkondensiert und führte eindeutig zu B-Tyr(SO$_3$Ba$_{1/2}$)-Thr(tBu)-Gly-Trp-Leu-Asp(OtBu)-Phe-NH$_2$ (III) mit 70% Ausbeute. Die hydrogenolytische Abspaltung des Benzyloxycarbonyl-restes von III unter üblichen Hydrierungsbedingungen ergab 96% Ausbeute an H-Tyr(SO$_3$Ba$_{1/2}$)-Thr(tBu)-Gly-Trp-Leu-Asp(OtBu)-Phe-NH$_2$ (IV). Eine anschließende Aufstockung des »aminofreien« Tyrosin-O-sulfat-Peptid-derivats IV mit B-Arg(B$_2$)-Asp(OtBu)-OH nach der DCCD/HOSU-Methode zu B-Arg(B$_2$)-Asp(OtBu)-Tyr(SO$_3$Ba$_{1/2}$)-Thr(tBu)-Gly-Trp-Leu-Asp(OtBu)-Phe-NH$_2$ (V) verlief ebenfalls mit 96% Ausbeute. Die hydrogenolytische Abspaltung der drei Benzyloxycarbonyl-gruppen vom aminoendständigen Arginin-rest bei etwa pH 6 lieferte das gewünschte Nonapeptid-derivat VI, d. h. H-Arg-Asp(OtBu)-Tyr(SO$_3$H)-Thr(tBu)-Gly-Trp-Leu-Asp(OtBu)-Phe-NH$_2$ mit 83% Ausbeute.

Trotz der vielfach in der Literatur angegebenen beachtlichen Säureinstabilität von Tyrosin-O-sulfat und seiner Peptide gelang auch die letzte Stufe der Herstellung des CCK-PZ-Nonapeptid-analogons, d. h. die Abspaltung der Schutzgruppen auf tert.-Butanolbasis mit überraschend positivem Ergebnis. Die befürchtete gleichzeitige teilweise Spaltung der Sulfathalbester-gruppierung trat nicht auf. Für die Abspaltung der Schutzgruppen eignen sich die hierfür bekannten sauren Mittel, bevorzugt wird etwa 70- bis etwa 90%ige Trifluoressigsäure. Zweckmäßig wird dabei ein Kationenfänger, wie z. B. 2-Methylindol, zugesetzt.

Die folgenden Beispiele erläutern die Erfindung weiter.

Beispiel 1

B-Tyr(SO$_3$Ba$_{1/2}$)-O-Ba$_{1/2}$ · 3H$_2$O (I)

12,7 g (40,3 mMol) B-Tyr-OH in Pyridin werden mit 25,8 g (161,1 mMol) Pyridin-SO$_3$-Komplex versetzt. Nach Erwärmen der Suspension auf 60°C und Rühren bei dieser Temperatur bis sich der Komplex gelöst hat (ca. 1/2 Stunde) wird auf 0°C gekühlt und filtriert; das Filtrat wird im Vakuum eingeengt und nochmals filtriert, um den überschüssigen Komplex abzutrennen. Nun wird die Lösung mit Wasser verdünnt und zweimal mit Essigester extrahiert, die abgetrennte wäßrige Phase wird mit Stickstoff gesättigt und je nach Menge des oben zurückgewonnenen Komplexes mit 2 bis 3 Äquivalenten Bariumhydroxid versetzt.

Der Niederschlag wird abgesaugt und der Überschuß an Bariumhydroxid als BaCO$_3$ durch Einleiten von CO$_2$ entfernt. Das Absinken des pH unter 7 wird hierbei durch Zugabe von Pyridin verhindert.

Nach Filtration engt man die Lösung auf ca. 100 ml ein und fällt das Produkt mit Äthanol aus. Chromatographisch rein in n-BuOH/AcOH/H$_2$O/Essigester (3 : 1 : 1 : 5).

$[\alpha]_{546}^{26} : +22{,}7°$ bzw. $[\alpha]_{D}^{20} : +18{,}9°$ (c = 1, in DMF).

Ausbeute: 21,34 g (91% der Theorie).

C$_{17}$H$_{15}$NO$_8$SBa · 3H$_2$O (584,80)

| | | | |
|---|---|---|---|
| Berechnet: | C 34,92 | H 3,62 | N 2,39 | Ba 25,88 |
| Gefunden: | C 34,87 | H 3,10 | N 2,20 | Ba 23,30 |

Ber. Rückstand 39,9% (als BaSO$_4$)
Gef. Glührückstand 40,5% (als BaSO$_4$).

## Beispiel 2

### A. B-Tyr(So$_3$Ba$_{1/2}$)-Thr(tBu)-Gly-Trp-Leu-Asp(OtBu)-Phe-NH$_2$(III)

Zu einer Lösung von 0,28 g (0,48 mMol) B-Tyr(SO$_3$Ba$_{1/2}$)-O · 3H$_2$O in Dimethylformamid wird bei 0° C 0,62 ml (0,48 mMol) einer 0,77 n-HCl-Lösung in Dioxan zugetropft. Unter Rühren gibt man 0,35 g (0,40 mMol) H-Thr(tBu)-Gly-Trp-Leu-Asp(OtBu)-Phe-NH$_2$ · H$_2$O, 70 mg (0,52 mMol) 1-Hydroxybenzotriazol und letztlich bei −10° C 99 mg (0,48 mMol) Dicyclohexylcarbodiimid zu. Nach 6 Stunden bei −4°C und 6 Stunden bei Raumtemperatur wird das Lösungsmittel im Vakuum entfernt und der Rückstand mit Äther digeriert. Das Rohprodukt wird in Dimethylformamid aufgenommen; man filtriert vom Unlöslichen und fällt das Produkt mit Wasser aus. Das Produkt wird nochmals aus Methanol nach Abfiltration vom Unlöslichen mit Äther gefällt.

Chromatographisch rein in n-BuOH/AcOH/H$_2$O/Essigester (3 : 1 : 1 : 5); Schmelzpunkt: 168° C (Zers.):

$[\alpha]^{20}_{546} : -22{,}7°$ (c = 1, in Dimethylformamid), $[\alpha]^{20}_{D} : -18{,}8°$.

C$_{61}$H$_{78}$N$_9$O$_{16}$SBa$_{0,5}$ (1294,11)
  Berechnet:    C 56,62    H 6,08    N 9,79
  Gefunden:    C 56,64    H 6,21    N 9,87

### B. H-Tyr(SO$_3$Ba$_{1/2}$)-Thr(tBu)-Gly-Trp-Leu-Asp(OtBu)-Phe-NH$_2$/1H$_2$O · 1 DMF

4,07 g (3,14 mMol) B-Tyr(SO$_3$)-Thr(tBu)-Gly-Trp-Leu-Asp(OtBu)-Phe-NH$_2$/$^1$/$_2$Ba werden in Dimethylformamid in Gegenwart von Palladium wie üblich hydriert. Nach Entfernung des Katalysators wird das Lösungsmittel im Vakuum abgezogen und der Rückstand mit Äther verrieben.

Chromatographisch rein in n-BuOH/AcOH/H$_2$O/Essigester (3 : 1 : 1 : 5), Schmelzpunkt 178° C (Zers.);

$[\alpha]^{20}_{D} : -29{,}85°$ bzw. $[\alpha]^{20}_{546} : -36°$ (c = 1, in DMF).

Ausbeute: 3,72 g (96% der Theorie)

C$_{53}$H$_{72}$N$_9$O$_{14}$SBa$_{0,5}$ · 1H$_2$O · 1 DMF (1235,10)
  Berechnet:    C 54,46    H 6,61    N 11,34
  Gefunden:    C 54,70    H 6,73    N 11,34

### C. B-Arg(B$_2$)-Asp(OtBu)-Tyr(SO$_3$Ba$_{1/2}$)-Thr(tBu)-Gly-Trp-Leu-Asp(OtBu)-Phe-NH$_2$/ · 2H$_2$O

3 g (2,43 mMol) H-Tyr(SO$_3$Ba$_{1/2}$)-Thr(tBu)-Gly-Trp-Leu-Asp(OtBu)-Phe-NH$_2$/ · 1H$_2$O und 2,18 g (2,92 mMol) B-Arg(B$_2$)-Asp(OtBu)-OH werden in Dimethylformamid mit 0,36 g (3,16 mMol) N-Hydroxysuccinimid und bei −20° C mit 0,63 g (3,04 mMol) Dicyclohexylcarbodiimid versetzt. Nach 24 Stunden bei 4°C und 24 Stunden bei Raumtemperatur wird die Reaktionsmischung vom ausgefallenen Harnstoff abfiltriert und im Vakuum eingeengt. Der Rückstand wird zweimal aus Dimethylformamid-Äther umgefällt. Chromatographisch rein in n-BuOH/AcOH/H$_2$O/Essigester (3 : 1 : 1 : 5); Schmelzpunkt 190 bis 195° C.

$[\alpha]^{20}_{D} : -14{,}3°$ bzw. $[\alpha]^{20}_{546} : -17{,}3°$ (c = 1, in DMF).

### D. H-Arg-Asp(OtBu)-Tyr(SO$_3$H)-Thr(tBu)-Gly-Trp-Leu-Asp(OtBu)-Phe-NH$_2$

3,7 g (1,9 mMol) B-Arg(B$_2$)-Asp(OtBu)-Tyr(SO$_3$Ba$_{1/2}$)-Thr(tBu)-Gly-Trp-Leu-Asp(OtBu)-Phe-NH$_2$/ · 2H$_2$O wurden wie üblich in Gegenwart von Palladium unter Zusatz von 26,34 ml 0,143 n-HCl in MeOH (theor. 26,87 ml) bei pH 6 in Dimethylformamid hydriert.

Nach Entfernung des Katalysators wurde das Produkt mit triäthylaminhaltigem Wasser gefällt. Der Niederschlag wurde aus Dimethylformamid-Wasser umgefällt und sorgfältig mit Wasser gewaschen.

Chromatographisch rein in n-BuOH/AcOH/H$_2$O/Essigester (3 : 1 : 1 : 5), Schmelzpunkt 208° C (Zers.);

$[\alpha]^{20}_{546} : -25{,}5°$ bzw. $[\alpha]^{20}_{D} : -21{,}2°$ (c = 1, in DMF).

Ausbeute: 2,24 g (83% der Theorie).

C$_{67}$H$_{97}$N$_{14}$O$_{18}$S (1418,7)
  Berechnet:    C 56,72    H 6,89    N 13,82
  Gefunden:    C 56,63    H 6,92    N 13,47

Beispiel 3

Nach dieser Methode konnte ferner folgende Verbindung hergestellt werden:

H-Arg-Asp-Tyr(SO$_3$H)-Thr-Gly-Trp-Nle-Asp-Phe-NH$_2$:
Aminosäuren-Analyse (berechnete Werte in Klammern) des sauren Hydrolysats (6M HCl/110°C/ 24 Std. unter Zusatz von 2,5% Thioglykolsäure):
 Arg 1,00(1), Asp 1,92(2), Tyr 1,00(1), Thre 1,03(1), Gly 0,98(1), Trp 0,91(1),
 Nle 1,02(1), Phe 1,00(1);
 des AP-M Abbaus:
 Arg 1,00(1), Asp 1,96(2), Tyr(SO$_3$H) 0,98(1), Thr 1,05(1),
 Gly 1,00(1), Trp 1,00(1), Nle 1,01(1), Phe 1,00(1);
 dünnschichtchromatographisch (HPTLC-Fertigplatten Kieselgel 60, Merck AG, Darmstadt) einheitlich in n-Butanol/Essigsäure/Pyridin/Wasser (60 : 6 : 40 : 24); einheitlich nach Hochdruck-flüssigkeitschromatographie [Säule: μ-Bondapak C 18; Eluens: 29% Acetonitril + 71% 0,01 H Ammoniumacetat Puffer, ph 4,0; isokratisch] und trägerfreien Elektrophorese [Kammerelektrolyth: 0,1 M Ammoniumacetat Puffer, pH 8,3; Elektrodenabstand: 50 cm bei 1500 V]; Tyr/Trp = 0,0 (laut UV-Messung).

## Patentansprüche

1. N-Acyl-tyrosin-O-sulfat-bariumsalze.
2. Verfahren zur Herstellung der N-Acyl-tyrosin-O-sulfat-bariumsalze, dadurch gekennzeichnet, daß man ein N-acyliertes Tyrosin in einem polaren organischen Lösungsmittel mit überschüssigem Pyridin-SO$_3$ umsetzt, die erhaltene Lösung mit Wasser extrahiert und aus der wäßrigen Phase das Bariumsalz des N-Acyl-tyrosin-O-sulfats durch Zusatz einer löslichen Bariumverbindung ausfällt.
3. N-Carbobenzoxy-tyrosin-O-sulfat-bariumsalz.
4. N-t-Butyloxycarbonyl-tyrosin-O-sulfat-bariumsalz.
5. N-Fluorenyloxycarbonyl-tyrosin-O-sulfat-bariumsalz.
6. N-2-Nitrophenylsulfenyl-tyrosin-O-sulfat-bariumsalz.
7. Verwendung der N-Acyl-tyrosin-O-sulfat-bariumsalze zum Einbau der Tyrosin-O-sulfat-Gruppe bei der Synthese von Peptiden.

## Claims

1. N-acyl-tyrosine-O-sulphate-barium salts.
2. A method of producing N-acyl-tyrosine-O-sulphate-barium salts, characterized in that an N-acylated tyrosine is reacted with excess pyridine-SO$_3$ in a polar organic solvent, the solution obtained is extracted with water and the barium salt of the N-acyl-tyrosine-O-sulphate is caused to precipitate from the aqueous phase by addition of a soluble barium compound.
3. N-carbobenzoxy-tyrosine-O-sulphate-barium salt.
4. N-t-butyloxycarbonyl-tyrosine-O-sulphate-barium salt.
5. N-fluorenyloxycarbonyl-tyrosine-O-sulphate-barium salt.
6. N-2-nitrophenylsulphenyl-tyrosine-O-sulphate-barium salt.
7. The use of N-acyl-tyrosine-O-sulphate-barium salts for incorporating the tyrosine-O-sulphate group in the synthesis of peptides.

## Revendications

1. Sels de baryum d'O-sulfate de N-acyl tyrosine.
2. Procédé pour la préparation des sels de baryum d'O-sulfate de N-acyl tyrosine, caractérisé en ce qu'on fait réagir une tyrosine N-acylée dans un solvant organique polaire avec un excès de complexe SO$_3$-pyridine, on épuise la solution obtenue avec de l'eau et on sépare par précipitation de la phase aqueuse le sel de baryum de l'O-sulfate de N-acyl tyrosine par addition d'un composé soluble du baryum.
3. Sel de baryum d'O-sulfate de N-carbobenzoxy-tyrosine.
4. Sel de baryum d'O-sulfate de N-t-butyloxycarbonyl-tyrosine.
5. Sel de baryum d'O-sulfate de N-fluorényloxycarbonyl-tyrosine.
6. Sel de baryum d'O-sulfate de N-2-nitrophényl-sulfényl-tyrosine.
7. Utilisation des sels de baryum d'O-sulfate de N-acyl tyrosine pour l'introduction du groupe O-sulfate de tyrosine dans la synthèse de peptides.